(19) Europäisches Patentamt

European Patent Office

Office européen des brevets

(11) **EP 0 996 399 B1**

(12) **EUROPÄISCHE PATENTSCHRIFT**

(45) Veröffentlichungstag und Bekanntmachung des Hinweises auf die Patenterteilung:
**25.09.2002 Patentblatt 2002/39**

(51) Int Cl.7: **A61F 13/15**, A61L 15/60, D06M 15/263, D06M 15/285

(21) Anmeldenummer: **98902956.6**

(22) Anmeldetag: **09.01.1998**

(86) Internationale Anmeldenummer:
**PCT/DE98/00093**

(87) Internationale Veröffentlichungsnummer:
**WO 99/003435 (28.01.1999 Gazette 1999/04)**

(54) **WINDEL MIT EINEM PH-SENSITIVEN SUPERABSORBER**

DIAPER WITH A PH-SENSITIVE SUPERABSORBER

COUCHE PRESENTANT UN MATERIAU SUPERABSORBANT OU SENSIBLE AU pH

(84) Benannte Vertragsstaaten:
**AT DE FR GB IT NL**

(30) Priorität: **16.07.1997 DE 19730448**

(43) Veröffentlichungstag der Anmeldung:
**03.05.2000 Patentblatt 2000/18**

(73) Patentinhaber: **FRAUNHOFER-GESELLSCHAFT ZUR FÖRDERUNG DER ANGEWANDTEN FORSCHUNG E.V.**
**80636 München (DE)**

(72) Erfinder:
• **GROSS, Hans-Jürgen**
**D-45661 Recklinghausen (DE)**
• **WACK, Holger**
**D-44137 Dortmund (DE)**
• **ALTHAUS, Wilhelm**
**D-59439 Holzwickede (DE)**

(56) Entgegenhaltungen:
**EP-A- 0 693 508        WO-A-92/20735**
**DE-A- 2 107 241**

• **PATENT ABSTRACTS OF JAPAN vol. 016, no. 411 (C-0979), 31.August 1992 & JP 04 139206 A (KOHJIN CO LTD), 13.Mai 1992,**

**Beschreibung**

Die Erfindung betrifft eine wiederverwendbare Windel, die mit einer Flüssigkeit aufnehmenden Absorberkomponente versehen ist und sowohl für Kleinkinder, wie auch in der Kranken- und Altenpflege eingesetzt werden kann.

Bisher verwendete Einwegwindeln zeichnen sich durch hohe Flüssigkeitsaufnahme der Windel aus, die durch eine in der Windel enthaltene Superabsorberschicht, bestehend aus dreidimensional schwach vernetzten Polymerstrukturen, die in der Lage sind, ein vielfaches ihres eigenen Trockengewichtes an Flüssigkeit aufzunehmen, realisiert wird.

Solche Windelsysteme bestehen aus einer äußeren Kunststoffschicht als Trägerkomponente (PE) und einem hautfreundlichen Innenmaterial (Zellstofflies), das gute Flüssigkeit-Transporteigenschaften aufweist. In das Innenmaterial ist das Superabsorberkissen eingearbeitet, so daß ein direkter Hautkontakt vermieden wird. Die bekannten Windelsysteme sind Einwegartikel und müssen nach einmaligem Gebrauch deponiert oder thermisch entsorgt werden. Eine Kompostierung scheidet aufgrund der Kunststoffschicht der Trägerkomponente aus. Es wird also die Umwelt belastet und natürliche Ressourcen vergeudet.

Als Alternative können herkömmliche Windeln aus textilen Materialien eingesetzt werden, die beispielsweise durch Serviceunternehmen den jeweiligen Kunden gegebenenfalls geliefert und nach Gebrauch abgeholt, und im Anschluß bei entsprechend hohem Reinigungsaufwand mit hohem Wasserverbrauch erneut zur Verfügung gestellt werden. Diese herkömmlichen Windeln haben jedoch den Nachteil, der durch die Superabsorber beseitigt ist, daß sie wesentlich öfter gewechselt werden müssen, da das Speichervermögen für Flüssigkeit des textilen Materials im Gegensatz zu den Superabsorbern wesentlich geringer ist.

Dadurch tritt ein unangenehmes Nässegefühl und die Gefahr des Wundwerdens der Haut auf, so daß die Akzeptanz der wiederverwendbaren textilen Windeln beim Verbraucher relativ gering geworden ist.

Um diesem Trend entgegenzuwirken, ist in EP 0 693 508 A1 die Verwendung temperatursensitiver Polymerkompositionen u.a. für Windeln beschrieben. Ein solches Polymer, auch Hydrogel genannt, hat die Fähigkeit eine Flüssigkeit bei bestimmten Temperaturen aufzunehmen und zu speichern und bei Überschreiten einer bestimmten Temperatur die aufgenommene Flüssigkeit wieder abzugeben. Das bedeutet, daß eine entsprechend benutzte Windel oberhalb dieser Temperatur erwärmt werden muß, um die Flüssigkeit wieder abzugeben um so eine Wiederverwendung zu ermöglichen. Dabei muß generell beachtet werden, daß bei Unterschreiten dieser Umschlagstemperatur kein weiterer Kontakt mit einer Flüssigkeit erfolgen sollte, da in diesem Fall entsprechende Flüssigkeit wieder aufgenommen wird und in einer nachfolgenden Trocknung wieder entfernt werden muß.

Es ist daher Aufgabe der Erfindung, Windeln mit hohem Flüssigkeitsaufnahmevermögen zur Verfügung zu stellen, die außerdem mehrfach verwendet werden können.

Erfindungsgemäß wird diese Aufgabe mit den Merkmalen des Patentanspruchs 1 gelöst. Vorteilhafte Ausgestaltungsformen und Weiterbildungen der Erfindung ergeben sich mit der Anwendung, der in den untergeordneten Ansprüchen enthaltenen Merkmale.

Der erfindungsgemäßen Windel wird als Absorberkomponente ein pH-sensitives Hydrogel verwendet, das in der Lage ist, durch pH-Wertänderung die aufgenommene Flüssigkeit wieder abzugeben, so daß eine Regeneration der Absorberkomponente möglich wird.

Der Phasenübergangspunkt, bei der die aufgenommene Flüssigkeit aus dem gequollenem Hydrogel wieder abgegeben werden kann, und die Sensitivität auf pH-Wertänderung kann durch die Wahl der Syntheseparameter (Temperatur, Eduktkonzentration, u.a.) und verschiedene einzusetzende Grundmonomere gezielt beeinflußt werden.

Die erfindungsgemäß zu verwendenden pH-sensitiven Hydrogele sollten jedoch bevorzugt bei einer pH-Werterhöhung und dies besonders bevorzugt bei pH-Werten oberhalb 7 die aufgenommene Flüssigkeit wieder abgeben und bei pH-Werten unterhalb 7 quellfähig sein. Der Regenerierungsprozeß kann dann beispielsweise bei einem Waschvorgang in einer herkömmlichen Waschmaschine ohne weiteres durchgeführt werden, wobei sich im Nachgang die relativ kurze Zeit, die für die Trocknung erforderlich ist, insbesondere in einer Energieeinsparung vorteilhaft auswirkt.

Die erfindungsgemäße Windel kann nach Art eines Baukastensystems aus drei verschiedenen Komponenten, nämlich der bereits definierten Absorberkomponente, einer Tägerkomponente und einer Einlagenkomponente bestehen, die bei Bedarf direkt vom Verbraucher zu einem Windelsystem zusammengesetzt werden können.

Die Trägerkomponente kann hierfür, wie dies aus dem Stand der Technik bekannt ist, aus einem Kunststoffmaterial bestehen, an dem Befestigungselemente, wie z.B. Klettverschlüsse vorhanden sind und die Trägerkomponente mit einem elastischem Material an den Bünden versehen ist. In diese Trägerkomponente können dann die Einlagekomponente und darunter die erfindungsgemäße Absorberkomponente, z.B. als Absorberkissen eingelegt werden, so daß ein direkter Hautkontakt der Absorberkomponente vermieden wird. Dies führt zu einem angenehmen Gefühl, da die abgesonderte Flüssigkeit weitestgehend vom Hydrogel aufgenommen wird und die Einlagekomponente relativ trocken bleibt. die Einlagekomponente relativ trocken bleibt.

Es besteht aber auch die Möglichkeit, die Absorberkomponente mit der Trägerkomponente dauerhaft zu verbinden, wobei die Verbindung durch Polymerisation erfolgen kann.

Es ist aber auch eine Kombination von Einlagekomponente und Absorberkomponente denkbar, bei der die Absorberkomponente von der Einlagekomponente umschlossen ist, so daß die Regeneration der Absorberkomponente und die Reinigung der Einlagekomponente bei einem gemeinsamen Waschvorgang erfolgen kann. Dabei kann es günstig sein, die Einlagekomponente, ähnlich wie ein Kissen auszubilden, in das die Absorberkomponente eingesteckt werden kann.

Die Absorberkomponente kann aber auch in Form eines Gelflieses eingesetzt werden oder es kann sich bei der Absorberkomponente um auf Fasern applizierte Gele handeln, wobei auch andere äquivalente Lösungen in Betracht gezogen werden können.

Das Hydrogel kann aus mindestens einem Monomer eines Acrylamides, eines Acrylates oder deren Derivaten hergestellt werden. Günstig ist es, ein solches Monomer als Basismonomer in Verbindung mit einem geeigneten Vernetzungsmonomer zu verwenden. Die gewünschten Eigenschaften können durch Verwendung eines zusätzlichen Comonomers zusätzlich gezielt beeinflußt werden.

Geeignete Gele können z.B. aus den in Tabelle 1 aufgeführten Monomersystemen hergestellt werden. So ist ein nichtionisches Gel unter der Bezeichnung NIPA erhältlich. Geeigneter sind ionische Gele, die unter den Bezeichnungen NIPA 5 % BCQ und NIPA 5 % MAP erhältlich sind. Die letztgenannten ionischen Gele haben ein höheres Flüssigkeitsaufnahmevermögen, als dies bei dem nichtionischen Gel der Fall ist.

Die erfindungsgemäßen Windeln haben gegenüber den bekannten Einwegwindeln den Vorteil, daß die einzelnen Komponenten jeweils mehrfach wieder verwendet werden können und außerdem eine getrennte Entsorgung möglich ist, so daß beispielsweise das Kunststoffmaterial recycelbar ist und die anderen Komponenten ohne weiteres kompostiert werden können. Demzufolge werden sowohl die Umwelt, wie auch die natürlichen Ressourcen weitestgehend geschont. Die erfindungsgemäße Windel vereint die Vorteile der Windeln nach dem Stand der Technik bei Vermeidung der Nachteile dieser Windeln.

Beispiel für ein temperatursensitives Gel:

Eduktlösung:

BM:     N-Isopropylacrylamid
VM:     N, N' -Methylenbisacrylamid
L:      vollentsalztes Wasser

Starter:

S 1:    Ammoniumpersulfat
S 2:    Natriumbisulfit

Konzentrationen:

$$\text{Vernetzergehalt} = \frac{\text{Masse (V) * 100}}{\text{Masse (BM)}} = 5\ \%$$

$$\text{Gesamtmonomergehalt} = \frac{\text{Masse (BM + V) * 100}}{\text{Masse (L)}} = 10\ \%$$

$$\text{Startermenge} = \frac{\text{Masse (BM + CM)}}{1000}$$

Abkürzungen:

BM:     Basismonomer

CM:     Comonomer
L:      Lösungsmittel
s:      Starter
VM:     Vernetzer

Beispiel für ein pH-sensitives Gel:

Eduktlösung 1:

BM:     Methylmethacrylat
CM:     (2-Dimethylaminoethyl)methacrylat
VM:     Divinylbenzol
L:      Vollentsalztes Wasser

Starter:

S:      2,2'-Azobis(isobutyronitril)

Konzentrationen:

BM + CM = 100 mmol
BM = 86 mmol
CM = 14 mmol
VM = 1 Gew. %
S = 0,5 Gew. % pro 100 $cm^3$ VE-Wasser

Abkürzungen:

BM:     Basismonomer
CM:     Comonomer
L:      Lösungsmittel
S:      Starter
VM:     Vernetzer

In der Tabelle 1 sind verschiedene Monomersysteme (Basis- und Comonomere) genannt, aus denen entsprechende thermosensitive und/oder pH-sensitive Hydrogele synthetisiert werden können.

Tabelle 1

| BM: | Basismonomer | CM: | Comonomer |
|-----|--------------|-----|-----------|
| BM  |              | **Acrylamid** | |
| CM  |              | 2-(Acryloyloxyl)ethylsäurephosphat | |
|     |              | 2-Acrylamido-2-methylpropansulfonsäure | |
|     |              | 2-Dimethylaminoethylacrylat | |
|     |              | 2,2'-Bis(acrylamido)essigsäure | |
|     |              | 3-(Methacrylamido)propyltrimethylammoniumchlorid | |
|     |              | Acrylamidomethylpropandimethylammoniumchlorid | |
|     |              | Acrylat | |

Tabelle 1   (fortgesetzt)

| BM:   Basismonomer | CM:   Comonomer |
|---|---|
| | Acrylonitril |
| | Acrylsäure |
| | Diallyldimethylammoniumchlorid |
| | Diallylammoniumchlorid |
| | Dimethylaminoethylacrylat |
| | Dimethylaminoethylmethacrylat |
| | Ethylenglykoldimethacrylat |
| | Ethylenglykolmonomethacrylat |
| | Methacrylamid |
| | Methylacrylamidopropyltrimethylammoniumchlorid |
| | N,N-Dimethylacrylamid |
| | N-(2(((5-(Dimethylamino)1-naphthalenyl)-sulfonyl)-amino)-ethyl)-2-acrylamid |
| | N(3-(Dimethylamino)propyl)acrylamidhydrochlorid |
| | N-(3-(Dimethylamino)propyl)methacrylamidhydrochlorid |
| BM | **Poly(diallyldimethylammoniumchlorid** |
| | Natrium 2-(2-Carboxylbenzoyloxy)ethylmethacrylat |
| | Natriumacrylat |
| | Natriumallylacetat |
| | Natriummethacrylat |
| | Natriumstyrolsulfonat |
| | Natriumvinylacetat |
| | Triallylamin |
| | Trimethyl(N-Acryloyl-3-aminopropyl)ammoniumchlorid |
| | Triphenylmethan-leuco-derivate |
| | Vinyl-terminated-polymethysiloxan |
| BM | **N-(2-Ethoxyethyl)acrylamid)** |
| BM | **N-3-(Methoxypropyl)acrylamid** |
| BM | **N-(3-Ethoxypropyl)acrylamid** |
| BM | **N-Cyclopropylacrylamid** |
| BM | **N-n-Propylacrylamid** |
| BM | **N-(Tetrahydrofurfuryl)acrylamid** |
| BM | **N-Isopropylacrylamid** |
| CM | 2-(Diethylamino)ethylmethacrylat |
| | 2-(Dimethylamino)ethylmethacrylat |
| | 2-Acrylamido-2-methyl-1-propanesulfonacrylat |
| | Acrylsäure |
| | Acrylamid |
| | Alkylmethacrylat |
| | Bis(4-dimethylamino)phenyl)(4-vinylphenyl)methyl-leucocyanid |
| | Concanavalin A (Lecitin) |
| | Hexylmethacrylat |

Tabelle 1   (fortgesetzt)

| BM: | Basismonomer | CM: | Comonomer |
|---|---|---|---|

Laurylmethacrylat
Methacrylsäure
Methyacrylamidopropyltrimethylammoniunchlorid
n-Butylmethacrylat
Poly(tetrafluoroethylen)
Polytetramethylenetherglykol
Natriumacrylat
Natriummethacrylat
Natriumvinylsulfonat
Vinyl-terminated-polymethysiloxan

BM    **N,N'-Diethylacrylamid**
CM    Methyacrylamidopropyltrimethylammoniumchlorid
N-Acryloxysuccinimidester
N-tert.-Butylacrylamid
Natriummethacrylat

BM    **2-Dimethylaminoethylacrylat**
CM    2-Acrylamido-2-methylpropansulfonsäure
Acrylamid
Triallylamin

BM    **Acrylat**
CM    Acrylamid

BM    **(2-Hydroxyethyl)-methacrylat**
CM    Divinylbenzen
N,N-Dimethylaminoethylmethacrylat
Poly(oxytetramethylendimethacrylat)

BM    **(2-Hydroxypropyl)-methacrylat**
CM    Divinylbenzen
N,N=Dimethylaminoethylmethacrylat
Poly(oxytetramethylendimethacrylat)

BM    **(N-Hydroxymethyl)-acrylamid**
CM    Divinylbenzen
N,N-Dimethylaminoethylmethacrylat
Poly(oxytetramethylendimethacrylat)

BM    **Methylmethacrylat**
CM    Divinylbenzen
N,N-Dimethylaminoethylmethacrylat
**Poly(oxytetramethylendimethacrylat)**

BM    **Poly(2-hyroxyethylmethacrylat)**

BM    **Poly(2-hydroxlproylmethacrylat)**

BM    **Polyethylenglykolmethacrylat**

Tabelle 1   (fortgesetzt)

| BM:   Basismonomer | CM:   Comonomer |
|---|---|
| BM | **Acrylsäure** |
| CM | **Methacrylamidopropyltrimethylammoniumchlorid** |
| BM | **Collagen** |
| BM | **Dipalmitoylphosphatidylethanolamin** |
| BM | **Poly-(4,6-decadien-1.10-diol-bis(n-butoxycarbonylmethylurethan))** |
| BM | **Poly-(bis((aminoethoxy)ethoxy)phosphazen)** |
| BM | **Poly-(bis((butoxyethoxy)-ethoxy)phosphazen)** |
| BM | **Poly-(bis((ethoxyethoxy)-ethoxy)phosphazen)** |
| BM | **Poly-(bis((methoxyethoxy)-ethoxy)phosphazen)** |
| BM | **Poly-(bis(methoxyethoxy)phosphazen)** |
| BM | **Polydimethylsiloxan** |
| BM | **Polyethylenoxid** |
| BM | **Poly(ethylen-dimethylsiloxan-ethylenoxid)** |
| BM | **Poly(N-acrylopyrrolidin)** |
| BM | **Poly-(n,n-dimethyl-N-((methacryloyloxy)-ethyl)-N-(3-sulfopropyl)ammonium betain** |
| BM | **Polyvinylalkohol** |
| BM | **Polyvinylalkohol-vinylacetat** |
| BM | **Polyvinylmethylether** |
| BM | **Furan modifiziertes Poly(n-acetylethylenimin)** |
| CM | **Maleinimid modifiziertes Poly(n-acetylethylenimin)** |
| Vernetzermonomere | **N,N'-Methylenbiscacrylamid**<br>**Diallylamin**<br>**Diallylammoniumchlorid**<br>**Triallylamind**<br>**Triallylammoniumchlorid**<br>**Diallylweinsäurediamid**<br>**Divinylbenzol**<br>**Ethylenglykoldimethylacrylat** |

**EP 0 996 399 B1**

**Patentansprüche**

1. Windel mit einer Flüssigkeit aufnehmenden Absorberkomponente,
   **dadurch gekennzeichnet,**
   **daß** die Absorberkomponente ein pH-sensitives Hydrogel enthält.

2. Windel nach Anspruch 1,
   **dadurch gekennzeichnet, daß** das pH-sensitive Hydrogel pH-Wertänderungen von sauer/neutral nach alkalisch die aufgenommene Flüssigkeit abgibt.

3. Windel nach Anspruch 1 oder 2,
   **dadurch gekennzeichnet, daß** die Absorberkomponente mit einer Trägerkomponente und/oder einer Einlagekomponente verwendbar ist.

4. Windel nach einem der Ansprüche 1 bis 3,
   **dadurch gekennzeichnet, daß** die Absorberkomponente mit der Trägerkomponente durch Polymerisation dauerhaft verbindbar ist.

5. Windel nach einem der Ansprüche 1 bis 4,
   **dadurch gekennzeichnet, daß** die Absorberkomponente ein Gelvlies ist.

6. Windel nach einem der Ansprüche 1 bis 4,
   **dadurch gekennzeichnet, daß** die Absorberkomponente als auf Fasern appliziertes Gel ausgebildet ist.

7. Windel nach einem der Ansprüche 1 bis 6,
   **dadurch gekennzeichnet, daß** die Absorberkomponente von der Einlagekomponente umschlossen ist.

8. Windel nach einem der Ansprüche 1 bis 7,
   **dadurch gekennzeichnet, daß** die Absorberkomponente ein ionisch pH-sensitives Hydrogel ist.

9. Windel nach einem der Ansprüche 1 bis 8,
   **dadurch gekennzeichnet, daß** das Hydrogel aus mindestens einem Monomer eines Acrylamides, einem Acrylat oder deren Derivaten hergestellt ist.

10. Windel nach Anspruch 9,
    **dadurch gekennzeichnet, daß** das Hydrogel aus einem Monomer als Basismonomer mit einem Vernetzungsmonomer hergestellt ist.


**Claims**

1. Nappy with an absorber component which absorbs liquid,
   **characterised in that**
   the absorber component contains a pH-sensitive hydrogel.

2. Nappy according to claim 1,
   **characterised in that** the pH-sensitive hydrogel releases the absorbed liquid with pH-value alterations from acid/neutral to alkaline.

3. Nappy according to claim 1 or 2,
   **characterised in that** the absorber component can be used with a backing component and/or a lining component.

4. Nappy according to one of claims 1 to 3,
   **characterised in that** the absorber component can be permanently connected to the backing component by polymerisation.

5. Nappy according to one of claims 1 to 4,
   **characterised in that** the absorber component is a gel mat.

**6.** Nappy according to one of claims 1 to 4,
**characterised in that** the absorber component is configured as gel applied to fibres.

**7.** Nappy according to one of claims 1 to 6,
**characterised in that** the absorber component is surrounded by the lining component.

**8.** Nappy according to one of claims 1 to 7,
**characterised in that** the absorber component is an ionically pH-sensitive hydrogel.

**9.** Nappy according to one of claims 1 to 8,
**characterised in that** the hydrogel is produced from at least one monomer of an acrylamide, an acrylate or derivatives of these.

**10.** Nappy according to claim 9,
**characterised in that** the hydrogel is produced from a monomer as the basic monomer with a cross-linking monomer.

**Revendications**

**1.** Couche comportant un composant absorbant les liquides,
**caractérisée en ce que**
le composant absorbant contient un hydrogel sensible au pH.

**2.** Couche selon la revendication 1,
**caractérisée en ce que**
par suite de modifications du pH passant du domaine acide/neutre au domaine alcalin, l'hydrogel sensible au pH rejette le liquide absorbé.

**3.** Couche selon la revendication 1 ou 2,
**caractérisée en ce que**
le composant absorbant est utilisable avec un composant de support et/ou un composant intercalaire.

**4.** Couche selon l'une quelconque des revendications 1 à 3,
**caractérisée en ce que**
le composant absorbant peut être assemblé durablement avec le composant de support par polymérisation.

**5.** Couche selon l'une quelconque des revendications 1 à 4,
**caractérisée en ce que**
le composant absorbant est un non-tissé de gel.

**6.** Couche selon l'une quelconque des revendications 1 à 4,
**caractérisée en ce que**
le composant absorbant est constitué d'un gel appliqué sur des fibres.

**7.** Couche selon l'une quelconque des revendications 1 à 6,
**caractérisée en ce que**
le composant absorbant est entouré du composant intercalaire.

**8.** Couche selon l'une quelconque des revendications 1 à 7,
**caractérisée en ce que**
le composant absorbant est un hydrogel ionique sensible au pH.

**9.** Couche selon l'une quelconque des revendications 1 à 8,
**caractérisée en ce que**
l'hydrogel est préparé à partir d'au moins un monomère d'un acrylamide, d'un acrylate ou de leurs dérivés.

**10.** Couche selon la revendication 9,

**caractérisée en ce que**
l'hydrogel est préparé à partir d'un monomère en tant que monomère de base, avec un monomère de réticulation.